(19) Europäisches Patentamt
European Patent Office
Office européen des brevets

(11) Veröffentlichungsnummer: **0 236 919**
**A2**

(12) # EUROPÄISCHE PATENTANMELDUNG

(21) Anmeldenummer: **87103028.4**

(22) Anmeldetag: **04.03.87**

(51) Int. Cl.³: **C 07 D 213/54**
A 01 N 43/40

(30) Priorität: **13.03.86 DE 3608382**

(43) Veröffentlichungstag der Anmeldung:
**16.09.87 Patentblatt 87/38**

(84) Benannte Vertragsstaaten:
**AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Fest, Christa, Dr.**
**Im Johannistal 20**
**D-5600 Wuppertal 1(DE)**

(72) Erfinder: **Brandes, Wilhelm, Dr.**
**Eichendorffstrasse 3**
**D-5653 Leichlingen 1(DE)**

(72) Erfinder: **Hänssler, Gerd, Dr.**
**Am Arenzberg 58a**
**D-5090 Leverkusen 3(DE)**

(72) Erfinder: **Reinecke, Paul, Dr.**
**Steinstrasse 8**
**D-5090 Leverkusen 3(DE)**

(54) **Phenylsulfonyl-pyridinaldoxime.**

(57) Phenylsulfonyl-pyridinaldoxime der Formel (I)

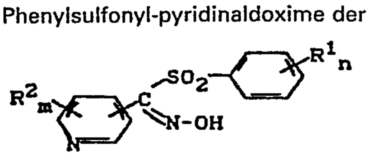

(I)

in welcher
$R^1$, $R^2$, n und m die in der Beschreibung gegebenen Bedeutungen haben sowie ihre Verwendung als Schädlingsbekämpfungsmittel.

Die neuen Phenylsulfonyl-pyridinaldoxime der Formel (I) können aus geeigneten Pyridinaldoximen mit Chlor und anschließendem Umsetzen mit geeigneten Sulfinsäuren bzw. deren Alkalisalzen hergestellt werden.

EP 0 236 919 A2

BAYER AKTIENGESELLSCHAFT 5090 Leverkusen, Bayerwerk
Konzernverwaltung RP
Patentabteilung

Bas/Kü-c
Ia

Phenylsulfonyl-pyridinaldoxime

Die vorliegende Erfindung betrifft neue Phenylsulfonyl-pyridinaldoxime, ein Verfahren zu ihrer Herstellung und ihre Verwendung als Schädlingsbekämpfungsmittel, insbesondere als Fungizide, und als Zwischenprodukte für weitere hochaktive Verbindungen.

Es sind bereits eine Reihe von Aldoxim-Derivaten bekannt. So z.B. Arylsulfonylbenzaldoxime, wie das α-Phenylsulfonyl-2,6-dichlorbenzaldoxim, und ihre Verwendung als Schädlingsbekämpfungsmittel, vor allem ihre Verwendung in Mitteln zur Bekämpfung von Weizensteinbrand, sind bekannt (vgl. Schweizer Patent Nr. 423.350).

Es wurden neue Phenylsulfonyl-pyridinaldoxime der allgemeinen Formel (I)

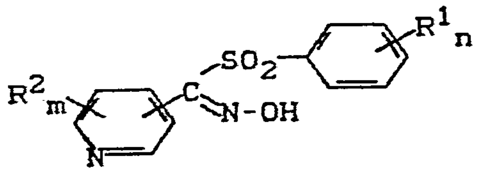

(I)

gefunden,

in welcher

$R^1$ für Halogen, Alkyl, Alkoxy, Alkylthio, Halogenalkyl, Halogenalkoxy, Halogenalkylthio, Nitro, Alkoxycarbonyl oder Alkylcarbonylamino steht,

$R^2$ für Halogen oder Alkyl steht,

n für eine ganze Zahl 0, 1, 2, 3, 4 oder 5 und

m für eine ganze Zahl 0, 1, 2, 3 oder 4 stehen,

wobei die Substituenten in den Ringen gleich oder verschieden sein können.

Weiterhin wurde gefunden, daß man die Phenylsulfonylpyridinaldoxime der allgemeinen Formel (I)

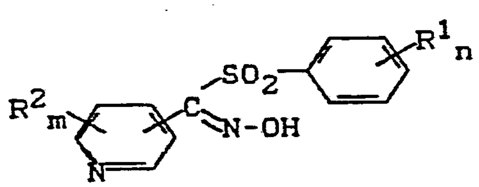

(I)

in welcher

$R^1$ für Halogen, Alkyl, Alkoxy, Alkylthio, Halogenalkyl, Halogenalkoxy, Halogenalkylthio, Nitro, Alkoxycarbonyl oder Alkylcarbonylamino steht,

$R^2$ für Halogen oder Alkyl steht,

n für eine ganze Zahl 0, 1, 2, 3, 4 oder 5 und

m für eine ganze Zahl 0, 1, 2, 3 oder 4 stehen,

wobei die Substituenten in den Ringen gleich oder verschieden sein können, erhält, wenn man Pyridinaldoxime der allgemeinen Formel (II)

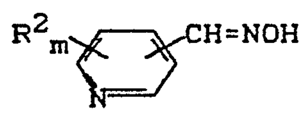

(II)

mit Chlor in einem Lösungsmittel zu α-Chlorpyridinaldoximen der Formel (IIa)

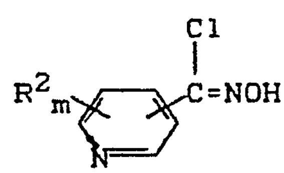

(IIa)

in welchen

$R^2$ und m die oben angegebenen Bedeutungen haben, umsetzt und diese anschließend mit Sulfinsäuren der allgemeinen Formel (III)

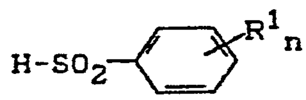

(III)

oder deren Alkalisalzen

in welcher

$R^1$ und n die oben angegebenen Bedeutungen haben,

gegebenenfalls in Gegenwart eines Lösungsmittels oder Verdünnungsmittels und gegebenenfalls in Gegenwart eines Säurebindemittels umsetzt.

Die erfindungsgemäßen Phenylsulfonylpyridinaldoxime der Formel (I) weisen starke biologische, vor allem fungizide Eigenschaften auf.

Überraschenderweise zeigen dabei die erfindungsgemäßen Verbindungen eine erheblich höhere, vor allem fungizide Wirksamkeit als die aus dem Stand der Technik bekannten Verbindungen, die strukturell und/oder wirkungsmäßig sehr naheliegende Verbindungen sind.

Die erfindungsgemäßen Verbindungen der Formel (I) können als syn- oder anti-Isomere oder als deren Gemische in

unterschiedlicher Zusammensetzung anfallen. Die Erfindung bezieht sich sowohl auf die reinen Isomeren als auch auf die Isomerengemische.

Die Alkylreste $R^1$ und $R^2$ sowie die Alkylteile in den Alkoxy-Resten in $R^1$ können geradkettig oder verzweigt sein und enthalten vorzugsweise jeweils 1 bis 6, insbesondere 1 bis 4 Kohlenstoffatome. Beispielhaft seien genannt: Methyl, Ethyl, n-Propyl, iso-Propyl, n-Butyl, sec.-Butyl, iso-Butyl, tert.-Butyl, n-Pentyl, iso-Pentyl, sec.-Pentyl, n-Hexyl, sec.-Hexyl, Methoxy, Ethoxy, n-Propoxy, iso-Propoxy, n-Butoxy, iso-Butoxy, sec.-Butoxy, tert.-Butoxy, n-Pentoxy, iso-Pentoxy, sec.-Pentoxy, n- Hexoxy und sec.-Hexoxy.

Die Alkylthio-Reste in $R^1$ können geradkettig oder verzweigt sein und enthalten vorzugsweise 1 bis 6, insbesondere 1 bis 4, besonders bevorzugt 1 bis 3 Kohlenstoffatome. Beispielhaft seien genannt: Methylthio, Ethylthio, n-Propylthio, iso-Propylthio, n-Butylthio, iso-Butylthio, sec.-Butylthio, tert.-Butylthio, n-Pentylthio und n-Hexylthio.

Die Halogenalkylteile in $R^1$ in den Resten Halogenalkyl, Halogenalkoxy und Halogenalkylthio enthalten vorzugsweise jeweils 1 bis 6, insbesondere 1 bis 4, besonders bevorzugt 1 oder 2 Kohlenstoffatome und vorzugsweise 1 bis 9, insbesondere 1 bis 5, besonders bevorzugt 1 bis 4 gleiche oder verschiedene Halogenatome. Beispielhaft seien genannt: Trichlormethyl, Trifluormethyl, Dichlorfluormethyl, Trichlorethyl, Tetrachlorethyl, Trichlormethoxy, Trichlorethoxy, Tetrachlorethoxy, Trichlormethylthio, Trifluormethylthio, Dichlorfluormethylthio, Trichlorethylthio, Tetrachlorethylthio.

Halogen in $R^1$ und $R^2$ auch in den Resten wie Halogenalkyl bedeutet Fluor, Chlor, Brom oder Iod, insbesondere Fluor oder Chlor, wenn an anderer Stelle nicht besonders definiert.

Alkoxycarbonyl und Alkylcarbonylamino in $R^1$ enthalten jeweils in den Alkylteilen vorzugsweis 1 bis 4, insbesondere 1 bis 3 Kohlenstoffatome, besonders bevorzugt 1 oder 2 Kohlenstoffatome. Beispielhaft seien genannt: Methoxycarbonyl, Ethoxycarbonyl, n-Propoxycarbonyl, iso-Propoxycarbonyl, n-Butoxycarbonyl, sec.-Butoxycarbonyl, iso-Butoxycarbonyl, tert.-Butoxycarbonyl, Methylcarbonylamino, Ethylcarbonylamino, n-Propylcarbonylamino, iso-Propylcarbonylamino, n-Butylcarbonylamino, iso-Butylcarbonylamino, tert.-Butylcarbonylamino und tert.-Butylcarbonylamino.

n bedeutet vorzugsweise 0, 1, 2, 3 oder 4, insbesondere 0, 1, 2 oder 3,

m bedeutet vorzugsweise 0, 1, 2 oder 3, insbesondere 0 oder 1.

Die erfindungsgemäßen Phenylsulfonyl-pyridinaldoxime sind durch die Formel (I) allgemein definiert. Bevorzugt sind Verbindungen der Formel (I), in welcher

$R^1$ für Halogen, Alkyl oder Alkoxy mit jeweils 1 bis 6 Kohlenstoffatomen, Alkylthio mit 1 bis 6 Kohlenstoffatomen, Halogenalkyl, Halogenalkoxy oder Halogenalkylthio mit jeweils 1 bis 6 Kohlenstoffatomen und jeweils 1 bis 9 gleichen oder verschiedenen Halogenatomen, Nitro, Alkoxycarbonyl oder Alkylcarbonylamino mit 1 bis 4 Kohlenstoffatomen im Alkoxy- oder Alkylteil steht,

$R^2$ für Halogen oder Alkyl mit 1 bis 6 Kohlenstoffatomen steht,

n für eine ganze Zahl 0, 1, 2, 3 oder 4 und

m für eine ganze Zahl 0, 1, 2 oder 3 stehen.

Besonders bevorzugt sind Verbindungen der Formel (I), in welcher

$R^1$ für Halogen, geradkettiges oder verzweigtes Alkyl mit 1 bis 4 Kohlenstoffatomen, geradkettiges oder verzweigtes Alkoxy mit 1 bis 4 Kohlenstoffatomen, geradkettiges oder verzweigtes Alkylthio mit 1 bis 4 Kohlenstoffatomen, Halogenalkyl mit 1 bis 4 Kohlenstoffatomen und 1 bis 5 gleichen oder verschiedenen Halogenatomen, Halogenalkoxy oder Halogenalkylthio mit jeweils 1 bis 4 Kohlenstoffatomen und jeweils 1 bis 5 gleichen oder verschiedenen Halogenatomen, Nitro, geradkettiges oder verzweigtes Alkoxycarbonyl mit 1 bis 3 Kohlenstoffatomen oder Alkylcarbonylamino mit 1 bis 3 Kohlenstoffatomen im Alkylteil steht,

$R^2$ für Halogen oder geradkettiges oder verzweigtes Alkyl mit 1 bis 4 Kohlenstoffatomen steht,

n für eine ganze Zahl 0, 1, 2, 3 oder 4 und

m für eine ganze Zahl 0, 1, 2 oder 3 stehen.

Insbesondere seien Verbindungen der Formel (I) genannt, in welchen

$R^1$ für Fluor, Chlor, geradkettiges oder verzweigtes Alkyl oder Alkoxy mit jeweils 1 bis 4 Kohlenstoffatomen, geradkettiges oder verzweigtes Alkylthio mit 1 bis 3 Kohlenstoffatomen, Halogenalkyl, Halogenalkoxy oder Halogenalkylthio mit jeweils 1 oder 2 Kohlenstoffatomen und jeweils 1 bis 4 gleichen oder verschiedenen Fluor- und Chloratomen, Nitro, Alkoxycarbonyl oder Alkylcarbonylamino mit jeweils 1 oder 2 Kohlenstoffatomen im Alkoxy- oder Alkylteil,

$R^2$ für Fluor, Chlor oder geradkettiges oder verzweigtes Alkyl mit 1 bis 4 Kohlenstoffatomen steht,

n für eine ganze Zahl 0, 1, 2, 3 oder 4 und

m für eine ganze Zahl 0, 1, 2 oder 3 stehen.

Ganz besonders bevorzugt seien die Verbindungen der Formel (I) genannt, in welchen

$R^1$ für Fluor, Chlor, Methyl, Ethyl, n-Propyl, iso-Propyl, Methoxy, Ethoxy, Methylthio, Trifluormethyl, Trifluormethoxy, Trifluormethylthio, Nitro, Methoxycarbonyl, Ethoxycarbonyl, Methylcarbonylamino oder Ethylcarbonylamino steht,

$R^2$ für Chlor, Methyl oder Ethyl steht,

n für eine ganze Zahl 0, 1, 2 oder 3 und

m für eine ganze Zahl 0 oder 1 stehen.

Verwendet man Pyrid-2-ylaldoxim, Chlor und 4-Methylphenylsulfinsaures Natrium als Ausgangsstoffe, so läßt sich der Reaktionsablauf des erfindungsgemäßen Verfahrens durch das folgende Formelschema darstellen:

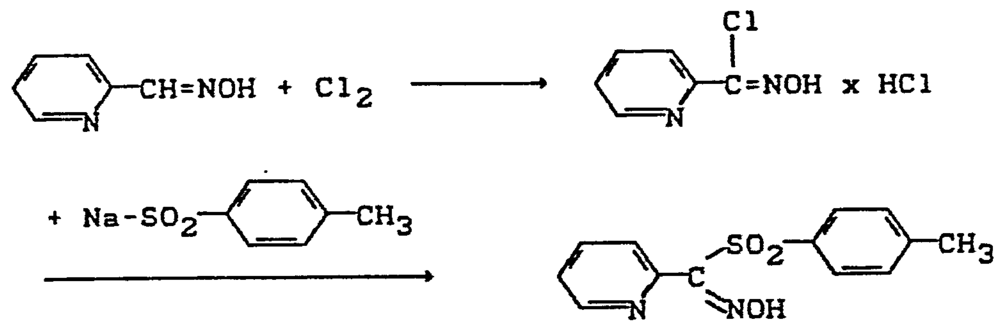

Die zur Durchführung des erfindungsgemäßen Verfahrens als Ausgangsstoffe benötigten Pyridinaldoxime sind durch die Formel (II) definiert. Diese Stoffe sind bekannt und können nach allgemein üblichen Verfahren hergestellt werden (siehe Houben Weyl, Methoden der organischen Chemie, Band XII, S. 1095ff).

Auch die Phenylsulfinsäuren der Formel (III) bzw. deren Alkalisalze sind bekannte Verbindungen der organischen Chemie (siehe Beilsteins Handbuch der organischen Chemie, Band II, S. 2 (4. Ausgabe).

Das erfindungsgemäße Verfahren kann gegebenenfalls in Gegenwart eines Lösungs- oder Verdünnungsmittels durchgeführt werden. Als solche kommen prinzipiell alle inerten organischen Lösungsmittel infrage. Bevorzugt verwendet man Kohlenwasserstoffe, gegebenenfalls chlorierte, wie z.B. Benzol, Toluol, Xylol, Methylenchlorid, Chloroform, Tetrachlorkohlenstoff, Chlorbenzol und Dichlorbenzol, ferner Ether wie Diethyl- und Dibutylether, Glykoldimethylether und Diglykoldimethylether, Tetrahydrofuran und Dioxan, weiterhin Ketone wie Aceton, Methylethyl-, Methylisopropyl- und Methylisobutylketon, außerdem Ester wie Essigsäure-methylester und -ethylester, ferner Nitrile wie z.B. Acetonitril und Propionitril, Benzonitril, Glutarsäuredinitril, darüber hinaus Amide wie z.B. Dimethylformamid, Alkohole wie z.B. Methanol, Ethanol und iso-Propanol.

Als Säurebindemittel kommen für das erfindungsgemäße Verfahren übliche anorganische oder organische Säurebinder in Frage. Als solche seien genannt: z.B. tert. Amine wie Triethylamin, Pyridin, Triethylendiamin u.a..

Die Reaktionstemperatur des erfindungsgemäßen Verfahrens kann in einem größeren Temperaturbereich variiert werden. Im allgemeinen arbeitet man bei der Chlorierung zwischen -20° C und 30° C, vorzugsweise zwischen -10° C und 20° C, während die 2. Stufe zwischen 0° C und 50° C, vorzugsweise zwischen 20° C und 30° C durchgeführt wird.

Die Reaktion wird normalerweise unter Normaldruck ausgeführt.

Bei der Durchführung des erfindungsgemäßen Verfahrens legt man im allgemeinen die Verbindungen der Formel (II) in einem Lösungsmittel vor und leitet trockenes Chlor ein bei -15° C, man arbeitet wie üblich auf und setzt die α-Chlorpyridinaldoxime der Formel (IIa) weiter um mit den Sulfinsäuren der Formel (III). Im allgemeinen werden die Komponenten (IIa) und (III) in äquimolaren Mengen eingesetzt oder die Sulfinsäuren im geringen Überschuß.

Die Aufarbeitung erfolgt nach allgemein üblichen Methoden.

Die erfindungsgemäßen Wirkstoffe weisen eine starke biologische Wirkung auf und können zur Bekämpfung von unerwünschten Schädlingen praktisch eingesetzt werden. Die Wirkstoffe sind u. a. für den Gebrauch als Schädlingsbekämpfungsmittel geeignet, vor allem als Fungizide.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;

Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielseise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die Wirkstoffe können in die üblichen Formulierungen übergeführt werden, wie Lösungen, Emulsionen, Suspensionen, Pulver, Schäume, Pasten, Granulate, Aerosole. Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie

Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine,

und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung

oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Die Verbindungen sind auch Ausgangsprodukte für weitere hochaktive Verbindungen. So kann man sie z.B. mit Chlorameisensäureestern umsetzen zu entsprechenden O-Alkoxy-Carbonylaldoximen, die ebenfalls gute Fungizide sind. Diese Verbindungen sind Gegenstand einer noch nicht veröffentlichten eigenen Anmeldung.

0236919

Anwendungsbeispiele

In den nachfolgenden Anwendungsbeispielen werden die nachstehend aufgeführten Verbindungen als Vergleichssubstanzen verwendet:

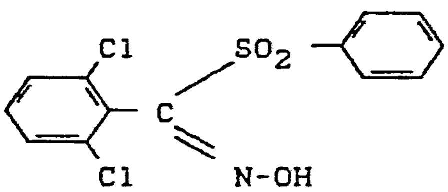

(A)

α-Phenylsulfonyl-2,6-dichlorbenzaldoxim und

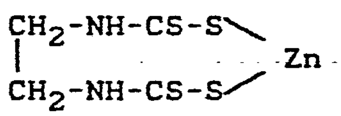

(B)

Zinkethylen-1,2-bis-dithiocarbamat

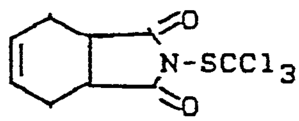

(C)

N-Trichlormethylthio-tetrahydrophthalimid

Beispiel A

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25$^0$C aufgestellt.

4 Tage nach der Inokulation erfolgt die Auswertung des Krankheitsbefalls.

Eine gute Wirksamkeit gegenüber dem Stand der Technik zeigen in diesem Test z.B. die Verbindungen gemäß den Herstellungsbeispielen: 7, 9, 8, 4, 6, 5, 1 und 11 bei einer Konzentration von 0,025.

Beispiel B

Venturia-Test (Apfel) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100 % relativer Luftfeuchtigkeit in einer Inkubationskabine.

Die Pflanzen werden dann im Gewächshaus bei 20°C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigt in diesem Test z.B. die Verbindung gemäß dem Herstellungsbeispiel 1.

Beispiel C

Leptosphaeria nodorum-Test (Weizen) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur con ca. 15°C und einer relativen Luftfeuchtigkeit von ca. 80 % aufgestellt.

10 tage nach der Inokulation erfolgt die Auswertung.

Eine deutliche Überlegenheit in der Wirksamkeit gegenüber dem Stand der Technik zeigen bei diesem Test z.B. die Verbindungen gemäß den Herstellungsbeispielen: 1, 4-9 und 11.

## Herstellungsbeispiele

### Beispiel 1:

Vorprodukt:

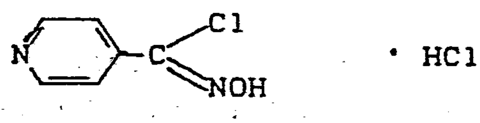

40,6 g (0,333 m) Pyridin-4-aldoxim werden in 350 ml Methylenchlorid gelöst und es wird bei -15°C Chlor (getrocknet) bis zur Sättigung eingeleitet. Man läßt das Reaktionsgemisch über Nacht bis auf Raumtemperatur kommen und saugt dann ab. Das Reaktionsprodukt wird mit Methylenchlorid, dann mit Aceton gewaschen und mit Ether nachgespült. Man erhält 60 g (93,4 % der Theorie) der gewünschten Substanz mit einem Schmelzpunkt von 203°C (Zersetzung).

Endprodukt:

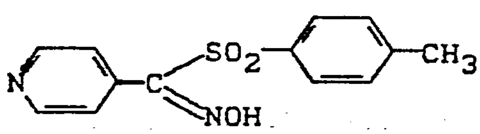

19,3 g (0,1 m) α-Chlor-pyridin-4-aldoxim Hydrochlorid werden in 250 ml Methanol gelöst und mit 18,7 g (0,105 m) 4-Methylphenylsulfinsaurem Natrium versetzt. Zur Freisetzung des α-Chlorpyridin-4-aldoxims werden noch 14 ml Triethylamin (0,1 m) hinzugefügt. Die Reaktion verläuft schwach

exotherm. Man hält das Reaktionsgemisch bei Zimmertemperatur und läßt bei dieser Temperatur über Nacht weiterrühren. Dann wird auf ca. 1 l Eiswasser gegossen, ausgerührt, abgesaugt, gewaschen und getrocknet. Das Reaktionsprodukt wird aus Isopropanol umkristallisiert. Man erhält 8,1 g (29 % der Theorie) der gewünschten Verbindung mit einem Schmelzpunkt von 138° C.

Analog Beispiel 1 können die Verbindungen der Formel (I)

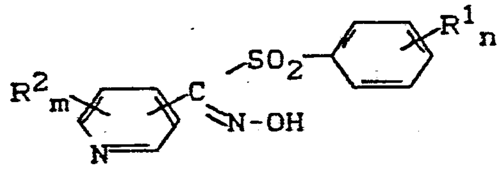 (I)

hergestellt werden:

| Beispiel Nr. | $R^1$ | $R^2$ | n | m | Stellung des Pyridinringes | Physikalische Daten (Schmelzpunkt °C) |
|---|---|---|---|---|---|---|
| 2 | - | - | 0 | 0 | 4 | 151 |
| 3 | 4-Cl | - | 1 | 0 | 4 | 125 |
| 4 | 4-$CH_3$ | - | 1 | 0 | 3 | 140 (Zers.) |
| 5 | - | - | 0 | 0 | 3 | 126 (Zers.) |
| 6 | 4-Cl | - | 1 | 0 | 3 | 139 (Zers.) |
| 7 | 4-$CH_3$ | - | 1 | 0 | 2 | 133 (Zers.) |
| 8 | - | - | 0 | 0 | 2 | 113 (Zers.) |
| 9 | 4-Cl | - | 1 | 0 | 2 | 131 |
| 10 | 4-$CH_3$ | 6-$CH_3$ | 1 | 1 | 2 | 142 |
| 11 | - | 6-$CH_3$ | - | 1 | 2 | 128 |
| 12 | 4-$OCH_3$ | - | 1 | 0 | 2 | 142 (Zers.) |
| 13 | 3-$CF_3$ | - | 1 | 0 | 2 | 120 (Zers.) |
| 14 | 4-F | - | 1 | 0 | 2 | 123 (Zers.) |

Beispiel A

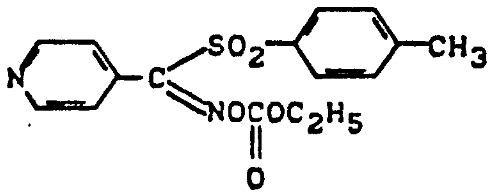

13,8 g (0,05 m) α-(4-Methylphenylsulfonyl)-pyridin-4-aldoxim werden in 150 ml Acetonitril gelöst und mit 7 ml (0,05 m) Triethylamin und 5,5 g (0,05 m) Chlorameisensäureethylester versetzt. Die Reaktion verläuft exotherm.

Das Reaktionsgemisch wird über Nacht bei Raumtemperatur gerührt, anschließend auf 750 ml Wasser gegossen, ausgerührt, abgesaugt, gewaschen und getrocknet. Das Reaktionsprodukt wird aus Isopropanol umkristallisiert. Man erhält 7,4 g (43 % der Theorie) der gewünschten Verbindung mit einem Schmelzpunkt von 126°C.

0236919

## Patentansprüche

1. Phenylsulfonyl-pyridinaldoxime der allgemeinen Formel (I)

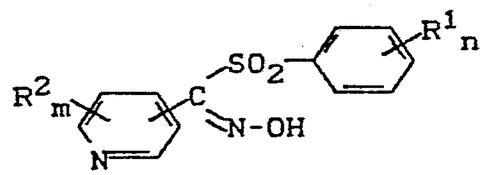

(I)

in welcher

$R^1$ für Halogen, Alkyl, Alkoxy, Alkylthio, Halogenalkyl, Halogenalkoxy, Halogenalkylthio, Nitro, Alkoxycarbonyl oder Alkylcarbonylamino steht,

$R^2$ für Halogen oder Alkyl steht,

n für eine ganze Zahl 0, 1, 2, 3, 4 oder 5 und

m für eine ganze Zahl 0, 1, 2, 3 oder 4 stehen.

2. Phenylsulfonyl-pyridinaldoxim-Derivate gemäß Anspruch 1, wobei in der Formel (I)

$R^1$ für Halogen, Alkyl oder Alkoxy mit jeweils 1 bis 6 Kohlenstoffatomen, Alkylthio mit 1 bis 6 Kohlenstoffatomen, Halogenalkyl, Halogenalkoxy oder Halogenalkylthio mit jeweils 1 bis 6 Kohlenstoffatomen und jeweils 1 bis 9 gleichen oder verschiedenen Halogenatomen, Nitro, Alkoxycarbonyl oder Alkylcarbonylamino mit 1 bis 4 Kohlenstoffatomen im Alkoxy- oder Alkylteil steht,

$R^2$ für Halogen oder Alkyl mit 1 bis 6 Kohlenstoffatomen steht,

n für eine ganze Zahl 0, 1, 2, 3 oder 4 und

m für eine ganze Zahl 0, 1, 2 oder 3 stehen.

3. Phenylsulfonyl-pyridinaldoxime gemäß Anspruch 1, wobei in der Formel (I)

$R^1$ für Halogen, geradkettiges oder verzweigtes Alkyl mit 1 bis 4 Kohlenstoffatomen, geradkettiges oder verzweigtes Alkoxy mit 1 bis 4 Kohlenstoffatomen, geradkettiges oder verzweigtes Alkylthio mit 1 bis 4 Kohlenstoffatomen, Halogenalkyl mit 1 bis 4 Kohlenstoffatomen und 1 bis 5 gleichen oder verschiedenen Halogenatomen, Halogenalkoxy oder Halogenalkylthio mit jeweils 1 bis 4 Kohlenstoffatomen und jeweils 1 bis 5 gleichen oder verschiedenen Halogenatomen, Nitro, geradkettiges oder verzweigtes Alkoxycarbonyl mit 1 bis 3 Kohlenstoffatomen oder Alkylcarbonylamino mit 1 bis 3 Kohlenstoffatomen im Alkylteil steht,

$R^2$ für Halogen oder geradkettiges oder verzweigtes Alkyl mit 1 bis 4 Kohlenstoffatomen steht,

n für eine ganze Zahl 0, 1, 2, 3 oder 4 und

m für eine ganze Zahl 0, 1, 2 oder 3 stehen.

4. Phenylsulfonyl-pyridinaldoxime gemäß Anspruch 1, wobei in der Formel (I)

$R^1$ für Fluor, Chlor, geradkettiges oder verzweigtes Alkyl oder Alkoxy mit jeweils 1 bis 4 Kohlenstoffatomen, geradkettiges oder verzweigtes Alkylthio mit 1 bis 3 Kohlenstoffatomen, Halogenalkyl, Halogenalkoxy oder Halogenalkylthio mit jeweils 1 oder 2 Kohlenstoffatomen und jeweils 1 bis 4 gleichen oder verschiedenen Fluor- und Chloratomen, Nitro, Alkoxycarbonyl oder Alkylcarbonylamino mit jeweils 1 oder 2 Kohlenstoffatomen im Alkoxy- oder Alkylteil,

$R^2$ für Fluor, Chlor oder geradkettiges oder verzweigtes Alkyl mit 1 bis 4 Kohlenstoffatomen steht,

n für eine ganze Zahl 0, 1, 2, 3 oder 4 und

m für eine ganze Zahl 0, 1, 2 oder 3 stehen.

5. Phenylsulfonyl-pyridinaldoxime gemäß Anspruch 1, wobei in der Formel (I)

$R^1$ für Fluor, Chlor, Methyl, Ethyl, n-Propyl, iso-Propyl, Methoxy, Ethoxy, Methylthio, Trifluormethyl, Trifluormethoxy, Trifluormethylthio, Nitro, Methoxycarbonyl, Ethoxycarbonyl, Methylcarbonylamino oder Ethylcarbonylamino steht,

$R^2$ für Chlor, Methyl oder Ethyl steht,

n für eine ganze Zahl 0, 1, 2 oder 3 und

m für eine ganze Zahl 0 oder 1 stehen.

6. Verfahren zur Herstellung von Phenylsulfonyl-pyridinaldoximen der allgemeinen Formel (I)

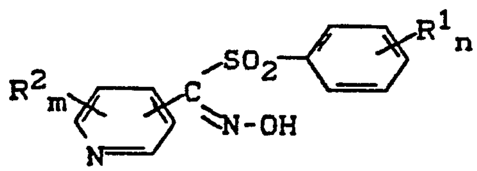

(I)

in welcher

$R^1$ für Halogen, Alkyl, Alkoxy, Alkylthio, Halogenalkyl, Halogenalkoxy, Halogenalkylthio, Nitro, Alkoxycarbonyl oder Alkylcarbonylamino steht,

$R^2$ für Halogen oder Alkyl steht,

n für eine ganze Zahl 0, 1, 2, 3, 4 oder 5 und

0236919

m für eine ganze Zahl 0, 1, 2, 3 oder 4 stehen, dadurch gekennzeichnet, daß man Pyridinaldoxime der allgemeinen Formel (II)

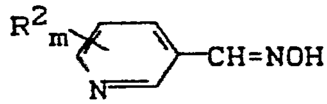 (II)

mit Chlor in einem Lösungsmittel zu α-Chlorpyridinaldoximen der Formel (IIa)

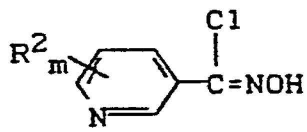 (IIa)

in welchen

$R^2$ und m die oben angegebenen Bedeutungen haben,

umsetzt und diese Verbindungen der Formel (IIa) anschließend mit Sulfinsäuren der allgemeinen Formel (III)

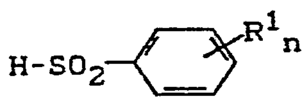 (III)

oder deren Alkalisalzen

in welcher

$R^1$ und n die oben angegebenen Bedeutungen haben,

gegebenenfalls in Gegenwart eines Lösungs- oder Verdünnungsmittels und gegebenenfalls in Gegenwart eines Säurebindemittels umsetzt.

7. Schädlingsbekämpfungsmittel, gekennzeichnet durch einen Gehalt an mindestens einem Phenylsulfonyl-pyridinaldoxim der Formel (I) gemäß den Ansprüchen 1 bis 6.

8. Verfahren zur Bekämpfung von Schädlingen, dadurch gekennzeichnet, daß man Phenylsulfonyl-pyridinaldoxime der Formel (I) nach den Ansprüchen 1 bis 6 auf Schädlinge und/oder ihren Lebensraum einwirken läßt.

9. Verwendung von Phenylsulfonyl-pyridinaldoximen der Formel (I) gemäß den Ansprüchen 1 bis 6 zur Bekämpfung von Schädlingen und als Zwischenprodukte.

10. Verfahren zur Herstellung von Schädlingsbekämpfungsmitteln, dadurch gekennzeichnet, daß man Phenylsulfonyl-pyridinaldoxime der Formel (I) gemäß den Ansprüchen 1 bis 6 mit Streckmitteln und/oder ober-flächenaktiven Mitteln vermischt.